# EUROPEAN PATENT APPLICATION

(11) **EP 3 680 668 A1**
(43) Date of publication of application: **15.07.2020**
(21) Application number: 20151048.4
(22) Date of filing: 09.01.2020
(51) Int. Cl.: G01N 33/68, G01N 33/574

(54) **EVALUATING SDHB PROTEIN EXPRESSION BY MASS SPECTROMETRY**

(30) Priority: 11.01.2019 US 201962791493 P
(71) Applicant: NantOmics, LLC, Culver City, CA 90232 (US)
(72) Inventor: KIM, Yeoun Jin, Culver City, CA 90232 (US); CHAMBERS, Andrew G., Culver City, CA 90232 (US)
(74) Representative: AWA Sweden AB

(57) **Abstract**

Methods are provided for detecting and quantifying expression of the SDHB protein. Specific protein fragment peptides are precisely detected and quantitated by DIA-mass spectrometry directly in tumor cells collected from tumor tissue that was obtained from a subject, such as a cancer patient. Results from this method can be used to aid choice of cancer therapy.

SEQUENCE LISTING

<110>  NantOmics,LLC

<120>  Evaluating SDHB Protein Expression by Mass Spectrometry

<130>  PAT.001281 // 17768-000092 // EP-21112701

<150>  62/791,493
<151>  2019-01-11

<160>  12

<170>  PatentIn version 3.5

<210>  1
<211>  12
<212>  PRT
<213>  Homo sapiens

<400>  1

Tyr Leu Gly Pro Ala Val Leu Met Gln Ala Tyr Arg
1               5                   10


<210>  2
<211>  11
<212>  PRT
<213>  Homo sapiens

<400>  2

Ile Tyr Pro Leu Pro His Met Tyr Val Ile Lys
1               5                   10


<210>  3
<211>  16
<212>  PRT
<213>  Homo sapiens

<400>  3

Ala Gly Asp Lys Pro His Met Gln Thr Tyr Glu Val Asp Leu Asn Lys
1               5                   10                  15


<210>  4
<211>  10
<212>  PRT
<213>  Homo sapiens

<400>  4

Gln Gln Tyr Leu Gln Ser Ile Glu Glu Arg
1               5                   10


<210>  5
<211>  10
<212>  PRT
<213>  Homo sapiens

<400>  5

Asn Glu Val Asp Ser Thr Leu Thr Phe Arg
1               5                   10

<210>  6
<211>  7
<212>  PRT
<213>  Homo sapiens

<400>  6

Ser Ile Glu Pro Tyr Leu Lys
1               5

<210>  7
<211>  7
<212>  PRT
<213>  Homo sapiens

<400>  7

Ile Asp Thr Asn Leu Asn Lys
1               5

<210>  8
<211>  14
<212>  PRT
<213>  Homo sapiens

<400>  8

Asp Leu Val Pro Asp Leu Ser Asn Phe Tyr Ala Gln Tyr Lys
1               5                   10

<210>  9
<211>  9
<212>  PRT
<213>  Homo sapiens

<400>  9

Leu Gln Asp Pro Phe Ser Leu Tyr Arg
1               5

<210>  10
<211>  7
<212>  PRT
<213>  Homo sapiens

<400>  10

Asp Asp Phe Thr Glu Glu Arg
1               5

<210>  11

<211>  8
<212>  PRT
<213>  Homo sapiens

<400>  11

Arg Ile Asp Thr Asn Leu Asn Lys
1               5


<210>  12
<211>  12
<212>  PRT
<213>  Homo sapiens

<400>  12

Ile Lys Asn Glu Val Asp Ser Thr Leu Thr Phe Arg
1               5                   10

## Description

### CROSS REFERENCE TO RELATED PATENT APPLICATIONS

This patent application claims the benefit of U.S. Provisional Patent Application No. 62/791,493 filed on 11 January 2019, the entire content of which is hereby incorporated by reference.

### FIELD

New and improved methods for measuring expression of the SDHB protein are provided. The methods use liquid chromatography-mass spectrometry (LC-MS) performed in the mode of Data Independent Acquisition (DIA). The methods allow detection of specific SDHB fragment peptides in cancer patient tumor tissue, and especially in gastrointestinal stromal tumors (GIST), and may be used as part of an improved method of cancer treatment. For example, the methods can be used to identify patients that will respond to, or are most likely to respond to, treatment with regimens that include but are not limited to imatinib, dasatinib, nilotinib, dacarbazine, and temozolomide.

### BACKGROUND

Succinate dehydrogenase iron-sulfur subunit, mitochondrial (SDHB), also known as iron-sulfur subunit of complex II (Ip), is a protein that, in humans, is part of the succinate dehydrogenase (also called SDH or Complex II) protein complex which catalyzes the oxidation of succinate (succinate + ubiquinone => fumarate + ubiquinol). SDHB is one of four protein subunits that together form succinate dehydrogenase, the other three being SDHA, SDHC and SDHD. The SDHB subunit is connected to the SDHA subunit on the hydrophilic, catalytic end of the SDH complex. It is also connected to the SDHC/SDHD subunits on the hydrophobic end of the complex anchored in the mitochondrial membrane. The SDHB subunit is an iron-sulfur protein with three iron-sulfur clusters.

Although the SDHB protein is expressed in the inner membrane of the mitochondria, the SDHB gene is nuclear, not mitochondrial. The expressed protein weighs 18.6 kDa and is composed of 180 amino acids. SDHB contains the iron-sulphur clusters necessary for tunneling electrons through the complex. It is located between SDHA and the two transmembrane subunits SDHC and SDHD The SDH complex is located on the inner membrane of the mitochondria and participates in both the Citric Acid Cycle and Respiratory chain. SDHB acts as an intermediate in the basic SDH enzyme. SDHB is a tumor suppressor gene and loss of the gene via mutation and/or loss of protein expression allows for the initiation and progression of tumorigenesis. Accordingly, a method of determining loss of protein expression would not only aid in potentially determining the level of tumorigenesis but also can be used in determining choice of therapy.

### SUMMARY

Methods are provided for detecting the presence of one or more fragment peptides from the SDHB protein by mass spectrometry utilizing the Data Independent Acquisition (DIA) mode of fragment peptide analysis. The fragment peptides can be the peptides of SEQ ID NOs:1-12, which are derived from the full-length SDHB protein.

A method for detecting expression of the SDHB protein in a biological sample of formalin fixed tissue is provided, including detecting and/or quantifying using mass spectrometry the amount of one or more fragment peptides derived from the SDHB protein in a protein digest prepared from the biological sample; and calculating the level of the protein in the sample. The fragment peptides may be selected from the peptides of SEQ ID NOs: 1-12, and may be any combination of 1 or more, 2 or more, 3 or more, 4 or more, 5 or more, 6 or more, 7 or more, 8 or more, 9 or more, 10 or more, 11 or more or all 12 of SEQ ID NOs: 1-12. The protein digest may optionally be fractionated prior to detecting and/or quantifying the amount of the one or more fragment peptides. The fractionating step may be, for example, liquid chromatography, nanoreversed phase liquid chromatography, high performance liquid chromatography, or reverse phase high performance liquid chromatography. The protein digest may be a protease digest, such as a trypsin digest and, advantageously, the protein digest of the biological sample may be prepared by the Liquid Tissue® protocol.

The tissue may be formalin-fixed tissue, and optionally may be paraffin-embedded. The tissue may be obtained from a tumor, such as a primary or secondary tumor.

The mass spectrometry may include one or more of tandem mass spectrometry, ion trap mass spectrometry, triple quadrupole mass spectrometry, ion trap/quadrupole hybrid mass spectrometry, MALDI-TOF mass spectrometry, MALDI mass spectrometry, and time of flight mass spectrometry. Advantageously, the mode of mass spectrometry used is Data Independent Acquisition (DIA).

The one or more fragment peptides may be quantified, for example by comparing an amount of the fragment peptide in one biological sample to the amount of the same fragment peptide in a different and separate biological sample. Detecting and/or quantifying the amount of at least one fragment peptide in the protein digest indicates the presence of SBHB protein and an association with the diagnostic stage/grade/status of cancer in the subject from whom the sample was taken. The detecting and/or quantifying the amount of the at least one fragment peptide, or the level of the corresponding protein may used as part of an improved method of cancer treatment therapy for the subject. The correlation may also may also be combined with detecting and/or quantifying the amount of other proteins or peptides from other proteins in a multiplex format to provide an improved method of cancer treatment for the subject.

### DETAILED DESCRIPTION

The succinate dehydrogenase (SDH) complex is a key respiratory enzyme composed of four subunits: SDHA, SDHB, SDHC and SDHD Remarkably, immunohistochemistry for SDHB becomes negative whenever there is bi-alleic inactivation of any component of SDH, which is very rare in the absence of syndromic disease. Therefore, loss of SDHB immunohistochemistry serves as a marker of syndromic disease, usually germline mutation of one of the SDH subunits. Tumors that show loss of SDHB expression are termed succinate dehydrogenase-deficient. In addition to loss of SDHB, tumors associated with SDHA mutation also show loss of SDHA expression. Fifteen per cent of pheochromocytoma and paraganglioma (PHEO/PGL) are associated with germline SDH mutation, and therefore SDH-deficient. SDH-deficient gastrointestinal stromal tumours (GISTs) show distinctive features, including absent KIT proto-oncogene receptor tyrosine kinase/platelet-derived growth factor receptor A (KIT/PDGFRA) mutations (but positive staining for cKIT and DOG1), virtually exclusive gastric location, lobulated growth, multi-focality, a prognosis not predicted by size and mitotic rate, frequent metastasis to lymph nodes and primary resistance to imatinib therapy. Thirty per cent are associated with SDHA germline mutation and 50% are associated with SDHC epimutation (post-zygotic promoter hypermethylation) - the hallmark of the syndromic but non-hereditary Carney triad (SDH-deficient GIST, SDH-deficient paraganglioma and pulmonary chondroma). SDH-deficient renal carcinoma shows vacuolated eosinophilic cytoplasmic and cytoplasmic inclusions. It is particularly associated with SDHB mutation, although SDHC and SDHA mutation occur. SDH-deficient pituitary adenomas are recognized, but appear to be the least common SDH-deficient neoplasm.

Paragangliomas are caused by SDHB mutations, demonstrate lack of SDHB protein expression, and have several other distinguishing characteristics. Malignancy is common, ranging from 38%-83% in mutation carriers. In contrast, tumors caused by SDHD mutations are almost always benign. Sporadic paragangliomas are malignant in less than 10% of cases. Malignant paragangliomas caused by SDHB are usually extra-adrenal (92% of all cases) while sporadic pheochromocytomas/paragangliomas are extra-adrenal in less than 10% of cases. The penetrance of the gene is often reported as 77% by age 50 (i.e. 77% of carriers will have at least one tumor by the age of 50). The average age of onset is approximately the same for SDHB vs non-SDHB related disease (approximately 36 years).

As with the SDHC and SDHD genes, SDHB is a tumor suppressor gene and loss of the gene via mutation and/or loss of protein expression allows for the initiation and progression of tumorigenesis. The SDHA gene is not a tumor suppressor gene.

Tumor formation generally follows the Knudson "two hit" hypothesis. The first copy of the gene is mutated in all cells, however the second copy functions normally. When the second copy mutates in a certain cell due to a random event, Loss of Heterozygosity (LOH) occurs and the SDHB protein is no longer produced. Tumor formation then becomes possible. Given the fundamental nature of the SDHB protein in all cellular function, it is not currently understood why only paraganglionic cells are affected. However, the sensitivity of these cells to oxygen levels may play a role.

The precise pathway leading from SDHB mutation to tumorigenesis is not well understood but several mechanisms have been proposed. The first potential mechanism is when succinate-ubiquinone activity is inhibited and electrons that normally would transfer through the SDHB subunit to the Ubiquinone pool are instead transferred to O₂ to create Reactive Oxygen Species (ROS), such as superoxide. ROS accumulate and stabilize the production of HIF1-α. HIF1-α combines with HIF1-β to form the stable HIF heterodimeric complex, in turn leading to the induction of antiapoptotic genes in the cell nucleus. A second potential mechanism is that SDH inactivation can block the oxidation of succinate, starting the following cascade of reactions: 1) the succinate accumulated in the mitochondrial matrix diffuses through the inner and outer mitochondrial membranes to the cytosol, 2) under normal cellular function, HIF1-α in the cytosol is quickly hydroxylated by prolyl hydroxylase (PHD), and 3) HIF1-α stabilizes and passes to the cell nucleus where it combines with HIF1-β to form an active HIF complex that induces the expression of tumor causing genes.

The cellular pathways involving SDHB raise the possibility of a therapeutic treatment. Succinate build-up inhibits PHD activity. PHD action normally requires oxygen and α-ketoglutarate as cosubstrates and ferrous iron and ascorbate as cofactors. Succinate competes with α-ketoglutarate in binding to the PHD enzyme. Therefore, increasing α-ketoglutarate levels can offset the effect of succinate accumulation. However, α-ketoglutarate does not permeate cell walls efficiently and therefore it is necessary to create a cell permeating derivative (e.g. α-ketoglutarate esters). *In-vitro* trials show this supplementation approach can reduce HIF1-α levels, and may result in a therapeutic approach to tumors resulting from SDH deficiency.

Paraganglionic tissue is derived from the neural crest cells present in an embryo. Abdominal extra-adrenal paraganglionic cells secrete catecholamines that play an important role in fetal development. After birth these cells usually die, a process that is triggered by a decline in nerve growth factor (NGF) which initiates apoptosis (cell death). This cell death process is mediated by an enzyme called prolyl hydroxylase EglN3. Succinate accumulation caused by SDH inactivation inhibits the prolyl hydroxylase EglN3. The net result is that paranglionic tissue that would normally die after birth remains, and this tissue may be able to trigger paraganglioma/pheochromocytoma later. Thus, inhibition of the Citric Acid Cycle forces the cell to create ATP glycolytically in order to generate its required energy. The induced glycolytic enzymes could potentially block cell apoptosis.

Consideration of the mutational status of the SDHB gene in patient tumor cells is currently employed in clinical practice using genomic methods that are well-known in the art. However, knowing the nucleic acid sequence of the SDHB gene cannot determine the expression status of the SDHB protein. The presently described methods permit detecting expression of the expressed SDHB protein in patient tumor cells by detecting the presence of one or more of the fragment peptides such as SEQ ID NO: 1-12 by mass spectrometry in the DIA mode.

Mass spectrometry has become a valuable approach to analysis of proteins directly in patient tumor tissue by detecting specified fragment peptides from full length proteins. Multiple types of mass spectrometer instruments can be run in multiple modes whereby different aspects of protein-derived fragment peptides can be analyzed.

The mass spectrometry may include but is not limited to, for example, tandem mass spectrometry, Q-TOF mass spectrometry, ion trap mass spectrometry, triple quadrupole mass spectrometry, MALDI-TOF mass spectrometry, MALDI mass spectrometry, hybrid ion trap/quadrupole mass spectrometry and/or time of flight mass spectrometry. The mode of mass spectrometry is Data Independent Acquisition (DIA).

Liquid chromatography interfaced to tandem mass spectrometers (LC-MS/MS) allows targeted and discovery-based global identification of proteins/peptides, post-translational modifications, and isoform variants. A widely used mass spectrometry approach for obtaining a global protein profile of a protein lysate from patient tissue is label-free shotgun proteomics where the mass spectrometer is operated in a Data Dependent Acquisition (DDA) mode. In this method, the most intense precursor ions from a survey scan are isolated and fragmented to produce tandem mass spectra (MS/MS or MS2) which are then matched against a database of known sequences for peptide identification. However, shotgun proteomics suffers from two major drawbacks; the frequent occurrence of under-sampling and analyzing the MS/MS spectra outside of the elution peak. This leads to low reproducibility whereby only a small fraction of detectable peptides will be reliably identified and sequenced.

A complementary approach to shotgun proteomics is the targeted mass spectrometry approach which utilizes the mode of Selected Reaction Monitoring (SRM). This approach uses the capabilities of triple quadrupole mass spectrometers to filter and selectively monitor a specific molecular ion and their corresponding fragment ions generated by collisional dissociation. These precursor-fragment ion pairs, termed SRM transitions, are repeatedly measured and counted over time, enabling reproducible quantification of the target peptides. However, SRM suffers from the need to develop an assay for each peptide of interest, low throughput analysis, and limited ability to multiplex across hundreds to thousands of peptides.

Data-independent acquisition (DIA), capitalizes on the strengths of both the shotgun and targeted methods by combining the reproducibility of SRM with the extensive number of proteins/peptides identified in shotgun proteomics. The DIA method in general avoids the need of detecting individual precursor ions during the analysis since the MS/MS scans are collected systematically (independently without precursor information) throughout the acquisition process. Multiple DIA formats are in use and/or currently under development. The methods described herein are not limited to any particular DIA format.

The data creation of DIA is more flexible and simpler compared to DDA or SRM experiments. DIA collects all MS/MS scans irrespective of precursor ion selections from a survey scan or full MS scan, in which DDA necessitates. The predefinition of target lists, which SRM/PRM requires, is unnecessary for DIA experiment. A broad range of precursors and corresponding transitions can be extracted after the data procurement. Thus, in targeted proteomics, DIA aims at inclusive proteome-wide quantification using targeted data extraction strategy. However, when compared to SRM, DIA-based targeted methods in general provide lower sensitivity, specificity, and reproducibility as well as smaller dynamic range in protein quantification.

The DIA method was originally introduced using an LTQ-linear ion trap (LIT) mass spectrometer with application of a wide precursor isolation window (10 *m*/*z*) to perform sequential isolation and fragmentation of a predefined *m*/*z* range. Compared to the MS level quantification, the S/N ratio was found to be greatly improved (higher than 350%) with a good linear dynamic range. The applicability of ion extractions in MS/MS level of DIA quantification was also demonstrated. However, such low resolution DIA MS/MS with the wide precursor isolation window decreased the mass accuracy and the confidence in peptide identification, which potentially resulted in increasing the false positive discovery rate.

Other modified DIA have subsequently been introduced. MS^{E} can be effectively operated on a QqTOF instrument. The use of smaller isolation windows (2.5u) led to the improvement of protein identification though the overall data acquisition covering the whole target mass range required multiple injections (67 injections for 5 days). A much faster scanning ion trap MS (e.g., LTQ Orbitrap Velos MS) reduced the whole data acquisition time to ∼2 days. Introduction of a bench top Exactive MS was used to demonstrate the application of all-ion fragmentation (AIF), in which peptides were injected to HCD collision cell for fragmentation without precursor selection and the fragments were returned back to C-trap and analyzed through Orbitrap mass analyzer. The assignment of fragment ions to co-eluting precursor ions was facilitated by high resolution (100 000 at *m*/*z* 200) and high mass accuracy. This concept significantly decreases the duty cycle time, but introduces more interferences from AIF at a certain retention time. Another DIA approach was subsequently introduced and is termed extended data-independent acquisition (XDIA), which was performed on a different type of Orbitrap MS with the capability of electron transfer dissociation (ETD). When compared to DDA-based ETD analysis, the DIA-based ETD approach significantly increased the number of identified spectra (∼250%) and the number of unique peptides (∼30%), which could potentially facilitate the low-abundance PTM study.

Recently, significant improvement in DIA has been achieved along with development of fast scanning HR/AM instruments whereby a variation of DIA was demonstrated using QqTOF MS, termed SWATH, to conceptually refer the utilization of a broad isolation window (typically 25 *m*/*z*) consisting of multiplexed spectra. One key feature of using QqTOF MS is the fastest data acquisition rate. Another DIA strategy was introduced by the use of QqOrbi MS, in which a novel acquisition method, namely MSX, was incorporated to improve the instrumental speed, selectivity, and sensitivity.

Recent improvements in DIA data acquisition and processing have been aided by newer versions of Orbitrap MS instruments. The first one termed pSMART, which was implemented on Q Exactive MS, utilizes asymmetric isolation windows over the mass range: 5 Da window covering 400-800 *m*/*z,* 10 Da window covering 800-1000 *m*/*z,* and 20 Da window covering 1000-1200 *m*/*z.* In another DIA method termed wide selected-ion monitoring DIA (wiSIM-DIA), which was implemented on the new hybrid Q-HCD-Orbitrap-LIT MS (i.e., Orbitrap Fusion and Lumos), three HR/AM SIM scans with 200 Da isolation windows are used to cover all precursor ions of 400-1000 *m*/*z.* In parallel with each SIM scan, 17 sequential ion-trap MS/MS with 12-Da isolation windows were acquired to cover the associated 200-Da SIM mass range.

Different from the standard wide-window MS/MS-only DIA methods, for pSMART and wiSIM-DIA the MS1 data (i.e., HR/AM precursor data with a longer fill time and enough precursor ion data points across the LC elution profile) is used for sensitive detection and quantification, while the MS/MS data from the fast ion trap MS/MS scan is used only for peptide identification/confirmation. Compared to the standard DIA methods with a complete recording of all fragment ions of the detectable peptide precursors but with sophisticated data analysis, *p*SMART and wiSIM-DIA can provide only a smaller number of MS/MS spectra for the detectable precursors with relatively easier data analysis because the majority of the duty cycle time is used for generating the high-quality MS1 data. Accordingly, their sensitivity and precision are higher than those provided by the standard DIA methods, while their quantification accuracy (i.e., specificity or selectivity) could be somewhat lower than that from the standard DIA methods due to the increased chances for having co-eluting interference from MS1 than MS/MS.

Very recently, a novel DIA method, termed hyper reaction monitoring (HRM), was demonstrated that consists of comprehensive DIA acquisition on a Q Exactive MS (hybrid quadruple/ion trap) platform and target data analysis with retention-time-normalized (iRT) spectral libraries. HRM has been demonstrated to outperform shotgun proteomics both in the number of consistently identified peptides and in reliable quantification of different abundant proteins across multiple measurements. More effective bioinformatics tools for DIA analysis are currently in development and may be applied in the presently described method.

DIA spectra are highly multiplexed and thus more elaborate data interpretation algorithms are needed compared to DDA or SRM/PRM. Currently, three approaches are used to decipher DIA spectra. The first one is to construct pseudo-DDA spectra from DIA spectra, such as Demux, MaxQuant, XDIA processor, and Complementary Finder. Those reconstructed pseudo-DDA spectra are then processed through conventional search engine tools such as MSGF+, MaxQuant, MASCOT, or other spectra libraries. Some of the schemes implemented the use of chromatographic elution profiles to improve identification of peptides. The recent publication by Tsou et al. ("DIA-Umpire: comprehensive computational framework for data-independent acquisition proteomics," Nature Methods 12:258-264 (2015) described the development of a computational approach, termed DIA-Umpire. DIA-Umpire starts with a two dimensional (*m*/*z* and retention time) feature-detection algorithm to discover all possible precursor and fragment ion signals in MS and MS/MS data. Fragment ions are grouped with a precursor ion that has a correlation of LC elution peak and retention time at a peak apex. Generated pseudo-MS/MS spectra for each precursor-fragment group are then processed with conventional database search engine including the abovementioned tools. The other approach is to match multiplexed MS/MS to theoretical spectra of peptides (e.g., ProbIDtree, Ion Accounting, M-SPLIT, MixDB, and FT-ARM). The scoring algorithms are directly based on how many theoretical fragment ions of a peptide from sequence databases or spectral libraries are found on multiplexed spectra with a high mass accuracy. The first two approaches have greatly tackled identification of peptides from DIA spectra prior to further quantification. Freely-available automated or semi-automated tools such as Skyline, mProphet, OpenSWATH, and DI-ANA and two commercial software, PeakView® from AB/Sciex and Spectronaut™ from Biognosys, have been employed to process quantitative targeted DIA data using the targeted data extraction strategy.

In these methods the specified SDHB fragment peptides may comprise the amino acid sequences are set forth as SEQ ID NO:1 (YLGPAVLMQAYR), SEQ ID NO:2 (IYPLPHMYVIK), SEQ ID NO:3 (AGDKPHMQTYEVDLNK), SEQ ID NO:4 (QQYLQSIEER),SEQ ID NO:5 (NEVDSTLTFR), SEQ ID NO:6 (SIEPYLK), SEQ ID NO:7 (IDTNLNK), SEQ ID NO:8 (DLVPDLSNFYAQYK), SEQ ID NO:9 (LQDPFSLYR), SEQ ID NO:10 (DDFTEER), SEQ ID NO:11 (RIDTNLNK), and SEQ ID NO:12 (IKNEVDSTLTFR).

The tumor sample may be, for example, a cell, collection of cells, or a solid tissue. The tumor sample may be formalin fixed solid tissue, and may be paraffin embedded tissue.

Detecting and quantitating the specified SDHB fragment peptides can be combined with detecting and quantitating other peptides from other proteins in a multiplex format so that the treatment decision about which agent used for treatment is based upon specific levels of the specified SDHB fragment peptide, in combination with other peptides/proteins in the biological sample. When specified SDHB peptides are not detected and therefore the protein is not expressed, then the therapeutic strategy may include one or more anti-Kit agents.

In the methods for detecting SDHB fragment peptides, the method may further include the step of fractionating the protein digest prior to detecting and/or quantifying the amount of the fragment peptide.

In these methods detecting and/or quantifying the amount of a fragment peptide in the protein digest indicates the presence of the corresponding protein and an association with cancer in the subject, and the results may be correlated to the diagnostic stage/grade/status of the cancer. The correlating step may be combined with detecting and/or quantifying the amount of other proteins or peptides from other proteins in a multiplex format to provide additional information about the diagnostic stage/grade/status of the cancer.

After the measurement and, optionally, the correlating step, the patient from which the biological sample was obtained is administered a therapeutically effective amount of a therapeutic agent, where the therapeutic agent and/or amount of the therapeutic agent administered is based upon the amount of the fragment peptide or the level of the protein.

Specifically, diagnostic methods for detecting and measuring expression of SDHB in a tumor sample or samples from the patient are provided. The tumor sample is advantageously a formalin-fixed sample. Using a DIA assay that measures one or more specific SDHB peptide fragments, and particular characteristics about the peptide, the presence and amount of the SDHB protein in cells derived from formalin fixed paraffin embedded (FFPE) tissue is determined. The peptide fragments derive from the full-length SDHB protein; and the specific peptide sequences used for SDHB are selected from the peptides of SEQ ID NOs:1-12.

Detection and accurate quantitation of these peptides in digests of FFPE tissue is highly unpredictable, due to the random protein crosslinking that occurs during formalin fixation of proteins. Surprisingly, however, it has been found that these specific SDHB peptides can be reliably detected and quantitated in digests prepared from FFPE samples of tumor tissue. See, for example, U.S. Pat. App. No. 13/993,045, the content of which is hereby incorporated by reference in its entirety.

More specifically, this DIA assay can measure the peptides directly in complex protein lysate sample prepared from cells procured from patient tissue samples, such as formalin fixed cancer patient tissue. Methods of preparing protein samples from formalin-fixed tissue are described in U.S. Pat. No. 7,473,532, the content of which is hereby incorporated by reference in its entirety. The methods described in U.S. Pat. No. 7,473,532 may conveniently be carried out using Liquid Tissue® reagents and protocol available from Expression Pathology Inc. (Rockville, Md.).

The most widely and advantageously available form of tissue, and cancer tissue, from cancer patients is formalin fixed, paraffin embedded tissue. Formaldehyde/formalin fixation of surgically removed tissue is by far the most common method of preserving cancer tissue samples worldwide and is the accepted convention in standard pathology practice. Aqueous solutions of formaldehyde are referred to as formalin. "100%" formalin consists of a saturated solution of formaldehyde (about 40% by volume or 37% by mass) in water. A small amount of stabilizer, usually methanol, is added to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature.

Results from the DIA assay can be used to correlate accurate and precise relative quantitative levels of the SDHB protein within the specific cancer of the patient from whom the tissue was collected and preserved. In certain embodiments, the comparison of the tumor SDHB level is made relative to SDHB levels from healthy tissue taken from the same patient, particularly from the same organ or tissue source as the tumor tissue. In other embodiments, the comparison is made relative to a standardized "normal" SDHB level calculated from measurements in a variety of healthy patients, or from a subpopulation of healthy patients (e.g., males, age 55 to 60 patients, KSHV⁺ patients, etc) to which the tumor patient belongs. This not only provides diagnostic information about the cancer, but also permits a physician or other medical professional to determine appropriate therapy for the patient. In this case, utilizing this assay can provide information about specific levels of SDHB protein expression in cancer tissue from a patient and makes it possible to determine whether or not the patient will respond favorably to therapy with the anti-Kit agents that specifically inhibit the function of the Kit protein.

The most widely-used methodology presently applied to determine protein presence in cancer patient tissue, especially FFPE tissue, is immunohistochemistry (IHC). IHC methodology uses an antibody to detect the protein of interest. The results of an IHC test are most often interpreted by a pathologist or histotechnologist. This interpretation is subjective and does not provide quantitative data that are predictive of sensitivity to therapeutic agents that target specific oncoprotein targets.

Studies involving other IHC assays, such as the Her2 IHC test, suggest the results obtained from such tests may be wrong or misleading. This is likely because different laboratories use different rules for classifying positive and negative IHC status. Each pathologist running a test also may use different criteria to decide whether the results are positive or negative. In most cases, this happens when the test results are borderline, *i.e.* the results are neither strongly positive nor strongly negative. In other cases, tissue from one area of cancer tissue can test positive while tissue from a different area of the cancer tests negative.

Inaccurate IHC test results may mean that patients diagnosed with cancer do not receive the best possible care. If all or part of a cancer is positive for a specific target oncoprotein but test results classify it as negative, physicians are unlikely to implement the correct therapeutic treatment, even though the patient could potentially benefit from agents that target the oncoprotein. If a cancer is oncoprotein target negative but test results classify it as positive, physicians may use a specific therapeutic treatment, even though the patient is not only unlikely to receive any benefit but also is exposed to the agent's secondary risks. For example, methods of treatment are disclosed herein in which the SDHB levels are measured prior to starting a patient on treatment with a drug selected from the group consisting of imatinib, dasatinib, nilotinib, dacarbazine, temozolomide, and combinations thereof. In certain embodiments, the drug is administered to a patient in which the SDHB levels are substantially equal to or greater than the SDHB levels in a matched sample of healthy tissue from the same patient.

Thus, there is great clinical value in the ability to correctly evaluate quantitative levels of the SDHB protein in tumors so that the patient will have the greatest chance of receiving a successful treatment regimen while reducing unnecessary toxicity and other side effects.

In order to develop the DIA assay for the SDHB fragment peptides additional information beyond simply the peptide sequence needs to be utilized by the mass spectrometer. That additional information is used to direct and instruct the mass spectrometer, (e.g., a triple quadrupole mass spectrometer) to perform the correct and focused analysis of the specified fragment peptides. A DIA assay may be effectively performed on a hybrid quadrupole/ion trap mass spectrometer. That type of a mass spectrometer may be considered to be one of the most suitable instruments for analyzing many target peptides within a very complex protein lysate that may consist of hundreds of thousands to millions of individual peptides from all the proteins contained within a cell. The additional information provides the mass spectrometer, such as a hybrid quadrupole/ion trap mass spectrometer, with the correct directives to allow analysis of many target peptides within a very complex protein lysate. DIA assays also can be developed and performed on other types of mass spectrometer, including MALDI, ion trap, ion trap/quadrupole hybrid, or triple quadrupole instruments, but presently the most advantageous instrument platform for a DIA assay is often considered to be a hybrid quadrupole/ion trap instrument platform. The additional information about target peptides in general, and in particular about the specified fragment peptides, may include one or more of the mono isotopic mass of each peptide, its precursor charge state, the precursor m/z value, the m/z transition ions, and the ion type of each transition ion. The peptide sequences of the specified SDHB fragment peptides as described in this method are shown in Table 1.

**Table 1**

| **SEQ ID** | **Peptide Sequence** |
|---|---|
| SEQ ID NO: 1 | YLGPAVLMQAYR |
| SEQ ID NO: 2 | IYPLPHMYVIK |
| SEQ ID NO: 3 | AGDKPHMQTYEVDLNK |
| SEQ ID NO: 4 | QQYLQSIEER |
| SEQ ID NO: 5 | NEVDSTLTFR |
| SEQ ID NO: 6 | SIEPYLK |
| SEQ ID NO: 7 | IDTNLNK |
| SEQ ID NO: 8 | DLVPDLSNFYAQYK |
| SEQ ID NO: 9 | LQDPFSLYR |
| SEQ ID NO: 10 | DDFTEER |
| SEQ ID NO: 11 | RIDTNLNK |
| SEQ ID NO: 12 | IKNEVDSTLTFR |

Because both nucleic acids and proteins can be analyzed from the same Liquid Tissue® biomolecular preparation it is possible to generate additional information about disease diagnosis and drug treatment decisions from the nucleic acids in same sample upon which proteins were analyzed. For example, if the SDHB protein expression is detected, when assayed by the DIA method the data can provide information about the state of the cells and their potential for uncontrolled growth, choice of optimal therapy, and potential drug resistance. At the same time, information about the status of genes and/or the nucleic acids and proteins they encode (e.g., mRNA molecules and their expression levels or splice variations) can be obtained from nucleic acids present in the same Liquid Tissue® biomolecular preparation. Nucleic acids can be assessed simultaneously to DIA analysis of proteins, including the SDHB protein. In one embodiment, information about SDHB protein and/or one, two, three, four or more additional proteins may be assessed by examining the nucleic acids encoding those proteins. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including but not limited to, single base pair polymorphisms, transitions, transversions, or combinations thereof.

## Claims

1. A method for detecting expression of the succinate dehydrogenase iron-sulfur subunit, mitochondrial (SDHB) protein in a biological sample of formalin fixed tissue, the method comprising
detecting and/or quantifying an amount of one or more fragment peptides derived from the SDHB protein in a protein digest prepared from said biological sample using mass spectrometry; and calculating the level of said protein in said sample.

2. The method of claim 1, further comprising the step of fractionating said protein digest prior to detecting and/or quantifying the amount of said one or more fragment peptides.

3. The method of claim 2, wherein said fractionating step is selected from the group consisting of gel electrophoresis, liquid chromatography, capillary electrophoresis, nanoreversed phase liquid chromatography, high performance liquid chromatography, and reverse phase high performance liquid chromatography.

4. The method of any one of the preceding claims, wherein said protein digest comprises a protease digest, preferably a trypsin digest.

5. The method of any one of the preceding claims, wherein said mass spectrometry comprises tandem mass spectrometry, ion trap mass spectrometry, triple quadrupole mass spectrometry, ion trap/quadrupole hybrid mass spectrometry, MALDI-TOF mass spectrometry, MALDI mass spectrometry, and/or time of flight mass spectrometry.

6. The method of claim 5, wherein a mode of mass spectrometry used is Data Independent Acquisition (DIA).

7. The method of any one of the preceding claims, wherein said one or more fragment peptides are selected from the group consisting of SEQ ID NO:1-12.

8. The method of any one of the preceding claims, wherein the tissue is paraffin embedded tissue.

9. The method of any one of the preceding claims, wherein the tissue is obtained from a tumor, such as primary or secondary tumor.

10. The method of any one of the preceding claims, wherein quantifying said one or more fragment peptides comprises comparing an amount of said one or more fragment peptides in the biological sample to an amount of the same one or more fragment peptides in a different and separate biological sample.

11. The method of any one of the preceding claims, wherein detecting and/or quantifying the amount of said one or more fragment peptides in the protein digest indicates the presence of the corresponding protein and an association with a diagnostic stage/grade/status of cancer in a subject.

12. The method of claim 11, further comprising correlating results of said detecting and/or quantifying the amount of said one or more fragment peptides, or the amount of the corresponding protein, to informing an optimal cancer treatment therapy for the subject.

13. The method of claim 12, wherein correlating the results is combined with detecting and quantifying an amount of other proteins or peptides from other proteins in a multiplex format.

14. The method of any one of the preceding claims, wherein the amount of the one or more fragment peptides derived from the SDHB protein in the biological sample is greater than or substantially equal to an amount of the one or more fragment peptides derived from SDHB protein in a sample of healthy tissue from the same source as the biological sample.

15. The method of any one of the preceding claims, further comprising determining that the patient from which the biological sample is derived shall be administered an agent selected from the group consisting of imatinib, dasatinib, nilotinib, dacarbazine, temozolomide, and a combination thereof.
